# DEMANDE DE BREVET EUROPEEN

(11) **EP 4 268 854 A1**
(43) Date de publication de la demande: **01.11.2023**
(21) Numéro de dépôt: 22305637.5
(22) Date de dépôt: 28.04.2022
(51) Int. Cl.: A61L 27/06, A61F 2/46

(54) **DISPOSITIF DE REGENERATION OSSEUSE GUIDEE EN TITANE ET PROCEDE DE FABRICATION**

(71) Demandeur: Nammour, Joseph, 75004 Paris (FR); Develop, 75016 Paris (FR)
(72) Inventeur: NAMMOUR, Joseph, 75004 Paris (FR); KHOURY, Georges, 75016 Paris (FR)
(74) Mandataire: Novagraaf Technologies

(57) **Abrégé**

La présente invention se rapporte à un dispositif de régénération osseuse guidée, destiné à la reconstruction d'un défaut osseux buccal, composé d'une paroi lisse en titane éventuellement microperforée, et ayant une forme recouvrant ledit défaut osseux buccal.

La présente invention se rapporte également à un procédé de fabrication d'un dispositif de l'invention, comprenant une étape de construction du dispositif de l'invention en fonction d'une représentation tridimensionnelle obtenue par une technique d'imagerie dento-maxillaire du défaut osseux.

## Description

### Domaine technique

La présente invention se rapporte à un dispositif de régénération osseuse guidée, destiné à la reconstruction d'un défaut osseux buccal, ainsi qu'à un procédé de fabrication d'un tel dispositif.

Ainsi, la présente invention trouve des applications dans le domaine médical, et plus particulièrement le domaine de la chirurgie dentaire.

Dans la description ci-dessous, les références entre crochets ([]) renvoient à la liste des références présentée à la fin du texte.

### Etat de la technique

Afin de régénérer des tissus détruits et/ou disparus en raison de perte osseuse, il existe plusieurs techniques d'augmentation osseuse, comme les greffes d'apposition, les greffes d'interposition, l'expansion, la distraction, les greffes de sinus ou encore la régénération osseuse guidée (ROG).

La ROG a pour objet de recréer de l'os afin de pouvoir placer et/ou stabiliser un implant dentaire, ou de recréer une crête pour une prothèse inamovible. Classiquement, une membrane est placée entre l'os et la gencive, pendant 3 à 6 mois, avant de pouvoir utiliser l'implant ou réaliser la prothèse inamovible. La membrane utilisée forme une barrière physique, permettant d'une part d'empêcher la colonisation du défaut osseux par les tissus mous conjonctifs et épithéliaux, et d'autre part limitant l'accès à l'espace cicatriciel aux seules cellules à pouvoir ostéogénique. Ces membranes ont ainsi un triple rôle : prévenir la prolifération des cellules à partir de la muqueuse de recouvrement et favoriser la migration des cellules issues des espaces médullaires dans le caillot habitant le site, stabiliser la greffe osseuse et le caillot et stabiliser le comblement par biomatériau.

Dans cette optique, plusieurs types de membranes, résorbables ou non résorbables, sont utilisés, comme par exemple les membranes en polytétrafluoroéthylène (PTFE) ou en collagène résorbable. Les caractéristiques des membranes non résorbables sont surtout l'inertie biologique, la flexibilité, la stabilité chimique et la micro-porosité asymétrique. En revanche, elles nécessitent une fixation par des vis et une deuxième intervention pour la dépose.

Au contraire, les membranes résorbables présentent l'avantage de ne pas devoir être retirées après la régénération osseuse, ce qui évite une potentielle irritation des tissus pouvant se produire lors du retrait, si la membrane s'est liée au tissu environnant pendant la phase de cicatrisation. Toutefois, les membranes résorbables ont pour inconvénient une possible interférence entre la résorption/cicatrisation et la régénération osseuse, ainsi que la nécessité d'un matériau supportant la membrane.

Il existe donc un réel besoin d'un dispositif palliant ces défauts, en particulier permettant d'obtenir une modélisation optimale de la reconstruction, ainsi qu'un mimétisme et une symétrie des structures osseuse.

### Description de l'invention

Les Demandeurs ont conçu un dispositif répondant à ces objectifs.

En effet, le dispositif de l'invention permet une augmentation osseuse, tant verticale qu'horizontale, permettant de combler le défaut osseux, une modélisation optimale de la reconstruction, ainsi qu'un mimétisme et une symétrie des structures osseuses.

Ainsi, un premier objet de l'invention se rapporte à un dispositif de régénération osseuse guidée, destiné à la reconstruction d'un défaut osseux buccal, composé d'une paroi lisse en titane éventuellement microperforée, et ayant une forme recouvrant ledit défaut osseux buccal.

On entend par « paroi », au sens de la présente invention, toute paroi non résorbable susceptible de former une barrière physique pour le contrôle de la migration cellulaire lors d'une régénération osseuse guidée (ROG). La paroi étant totalement lisse, c'est-à-dire non rugueuse, sur les deux faces du dispositif, elle est totalement hermétique, ce qui limite le phénomène d'adhérence des muqueuses au dispositif. En outre, cela implique avantageusement que l'os ne va pas incorporer le dispositif une fois celui-ci mis en place, ce qui évite les complications lors de la dépose du dispositif sur le plan muqueux et/ou osseux.

La paroi peut avoir une épaisseur adaptée à sa fonction, c'est-à-dire permettre une protection du biomatériau le temps nécessaire à la reconstruction osseuse. A ce titre, l'homme du métier peut aisément déterminer l'épaisseur adaptée en fonction des cas. Par exemple, l'épaisseur du dispositif de l'invention peut être comprise entre 0,1 mm et 2,5 mm, par exemple être d'environ 0,1mm, ou 0,2mm, ou 0,3mm, ou 0,4mm, ou 0,5mm, ou 0,6 mm ou 0,7 mm ou 0,8 mm ou 0,9 mm ou 1,0 mm ou 1,1 mm ou 1,2 mm ou 1,3 mm ou 1,4 mm ou 1,5 mm ou 1,6 mm ou 1,7 mm ou 1,8 mm, ou 1,9 mm, ou 2,0 mm, ou 2,1 mm, ou 2,2 mm, ou 2,3 mm, ou 2,4 mm, ou 2,5 mm.

Avantageusement, le dispositif de l'invention a une forme tridimensionnelle recouvrant au moins en partie, et de préférence totalement, le défaut osseux. Ainsi, à l'issue de la reconstruction osseuse, le volume reconstruit peut être substantiellement identique à celui dû à la perte osseuse traitée, voire supérieur.

Afin d'obtenir une forme tridimensionnelle recouvrant la forme du défaut osseux à traiter, toute technique de modélisation du volume et/ou de la quantité osseuse à reconstruire peut être utilisée. Il peut s'agir d'une technique d'imagerie dento-maxillaire du défaut osseux, comme par exemple la tomographie volumique à faisceaux coniques (également appelée Cône beam). Avantageusement, le défaut osseux peut être schématisé numériquement sur un logiciel adapté pour quantifier la substance osseuse à régénérer, comme par exemple les logiciels MIMICS ou 3-matic, cette liste n'étant pas limitative.

Le dispositif de l'invention est ainsi fabriqué en fonction de la forme modélisée, de manière à mimer la planification 3D, par toute technique adaptée et connue de l'homme du métier, comme par exemple par moulage, par usinage ou par impression 3D. Eventuellement, à partir du schéma, une couche supplémentaire de 1 mm d'épaisseur peut être rajoutée à la représentation 3D, avant de construire le dispositif de l'invention, pour avantageusement pallier une formation épithéliale superficielle qui peut se produire dans certaines régénérations.

Avantageusement, le dispositif de l'invention possède une forme appropriée lui permettant de recouvrir tout ou une partie du défaut osseux et de permettre la reconstruction de tout le volume osseux perdu, si nécessaire. Avantageusement, quelle que soit la forme choisie, le dispositif ménage un espace creux, lors de son installation, entre la paroi de celui-ci et l'os résiduel, de manière à permettre l'introduction d'un biomatériau à l'intérieur du dispositif de l'invention. Toute forme permettant d'atteindre cet objectif peut être utilisée par l'homme du métier. Il peut s'agir par exemple d'une forme choisie parmi une coquille, une plaque rigide, coque, et un filet (également appelé mesh). Par contre, il ne peut s'agir d'une grille. Quelle que soit la forme utilisée, le dispositif peut être alvéolé ou non alvéolé (2 mm à 30 mm, ronde ou ovalloide, rectangulaire ou polylobée), dans le but de permettre le remplissage de l'espace creux ménagé entre l'os résiduel et le dispositif par des biomatériaux ou de l'os autologue.

Ainsi qu'énoncé précédemment, le dispositif de l'invention est composé de titane. Le titane peut être du titane pur, ou un alliage de titane composé de titane et d'au moins un autre élément chimique. Lorsqu'il s'agit d'un alliage, il peut s'agir de tout alliage de titane adapté à une utilisation médicale. Notamment, l'alliage de titane peut comprendre au moins un autre élément choisi parmi l'aluminium, le vanadium, le fer, l'afnium, le molybdène, l'oxygène, le palladium, l'étain, le niobium, le zirconium et le tantale. Il peut s'agir par exemple d'un alliage choisi parmi Ti-6Al-4V, Ti-6Al-7Nb, Ti-5Al-2,5Fe, Ti-13Ng-13Zr, Ti-12Mo-6Zr-2Fe (appelé également « TMZF »), Ti-15Mo-5Zr-3Al, Ti-15Mo-3Nb-30, Ti-15Zr-4Nb-2Ta-0.2Pd, Ti-15Sn-4Nb-2Ta-0.2Pd, Ti-35Nb-7Zr-5Ta (également appelé « TNZT »), Ti-29Nb-13Ta-4,6Zr, Ti-35Nb-5Ta-7Zr-0,4O (également appelé « TNZTO ») et Ti-Mo. La teneur du dispositif en titane peut être par exemple comprise entre 88% et 100%, par exemple 88%, ou 89%, ou 90% ou 91% ou 92% ou 93% ou 94% ou 95% ou 96%, ou 100%. Il peut s'agir par exemple d'une composition commerciale, comme par exemple des Ti Grade 1, 2, 3 ou 4. Avantageusement, le titane est utilisé dans le dispositif de l'invention pour ses propriétés physiques, comme sa résistance à la rupture et à la fissuration, son haut degré de biocompatibilté, sa neutralité, et le fait qu'il induise très peu d'inflammation. Avantageusement, le titane constitue une barrière cellulaire et tissulaire. De plus, il est avantageusement biocompatible et non résorbable et son retrait est facile. Grâce aux caractéristiques du dispositif de l'invention, le greffon est avantageusement stabilisé et compressé, et montre un niveau de résorption quasiment nul.

Avantageusement, le dispositif de l'invention peut comprendre au moins une perforation pour la stabilisation du dispositif, chaque perforation étant destinée à recevoir une vis d'ostéosynthèse. A ce titre, la ou les perforation(s) est/sont réalisée(s) sur les parois du dispositif. Le nombre de perforations peut être déterminé selon des critères classiques appliqués dans ce domaine, par exemple en fonction de la taille du défaut osseux. Il peut s'agir à ce titre d'un nombre de perforations compris entre 1 et 4, voire entre 1 et 20, voire plus, en fonction des besoins et du volume à reconstruire.

Avantageusement, le dispositif peut en outre éventuellement comprendre des microperforations sur tout ou partie de sa paroi. Le fait que le dispositif comprenne des microperforations n'empêche pas qu'il soit également lisse, car les microperforations ne rendent pas le titane rugueux. Il est en effet indispensable que le dispositif ne soit pas rugueux, afin d'éviter les problèmes lors de la dépose dus à des phénomènes d'adhérence muqueuse ou osseuse. Les microperforations peuvent être réparties sur ladite paroi de manière uniforme, ou de manière irrégulière. Les microperforations peuvent présenter un diamètre compris entre 10 µm et 800 µm, par exemple entre 20 µm et 700 µm, ou entre 50 µm et 600 µm, entre 100 µm et 500 µm, ou encore entre 150 µm et 300 µm. Avantageusement, cette gamme de diamètre des microperforations permet d'obtenir une exclusion cellulaire entre les compartiments osseux et muqueux, ce que ne permettent pas les dispositifs, et notamment les grilles, de l'état de la technique. La densité des microperforations sur la paroi peut être comprise entre 1 et 100 microperforations par centimètre carré, par exemple entre 20 et 90, ou entre 30 et 80. Les trous sont ordinairement ronds, mais ils peuvent être de toute autre forme compatible avec leur fonction, par exemple de forme carrée, ovale ou rectangulaire. Avantageusement, les microperforations peuvent permettre d'améliorer l'angiogenèse lors de la régénération des tissus. En effet, les microperforations peuvent permettre à la néovascularisation d'accéder au biomateriau à partir des tissus mous, en plus de celle qui arrive via l'os natif ou la crête native à augmenter. Les micro-perforations peuvent aussi améliorer l'adhérence des tissus mous sur la plaque de titane, évitant ainsi les tensions au niveau de la zone des sutures et diminuant les expositions initiales et tardives du dispositif. Les microperforations peuvent être réalisées sur le dispositif par tout procédé ou outil connu de l'homme du métier, par exemple au moyen d'une fraise fine ou d'un laser.

Avantageusement, le dispositif de l'invention peut comprendre au moins une fenêtre aménagée dans sa paroi, notamment destinée à l'insertion d'un biomatériau et sa condensation. Le biomatériau, une fois inséré entre l'os et la paroi du dispositif de l'invention, c'est-à-dire dans l'espace à reconstruire, va permettre une ostéoactivité et ainsi reconstruire l'os souhaité. Tout type de biomatériau adapté à la reconstruction osseuse peut être utilisé dans ce cadre, comme tout type de substitut osseux choisi parmi les substituts osseux allogéniques, xénogeniques, autogéniques et synthétiques, cette liste n'étant pas limitative. La taille, la forme et le nombre de fenêtres peut être choisi par l'homme du métier en fonction notamment de la taille du dispositif de l'invention, ainsi que de la nature des défauts osseux à traiter. Par exemple, une fenêtre peut être de forme carrée, ronde, ovale ou rectangulaire. Ses dimensions peuvent par exemple être inscrites dans un rectangle de 1 à 20 mm de haut et de 1 à 30 mm de large ou plus. Une fenêtre peut être dessinée selon toute technique adaptée connue de l'homme du métier, par exemple au moyen d'un planificateur 3D. Le nombre de fenêtres peut être compris entre 1 et 5, et peut être par exemple de 1, 2, 3, 4 ou 5. Toutefois, le nombre de fenêtres peut être adapté par l'homme du métier en fonction de ses besoins. La ou les fenêtres peuvent être stabilisées par un dispositif de fixation, comme une vis d'ostéosynthèse, des fils, des engrenages ou encore des emboitements. Avantageusement, la fonction d'une fenêtre est de permettre le comblement de la chambre ou de l'espace créé par le dispositif avec des biomatériaux, ainsi que le tassement de ces derniers lors de l'apport et lors de la fixation de la fenêtre.

Avantageusement, le dispositif de l'invention est facile à retirer une fois le temps de pose nécessaire à la reconstruction osseuse atteint. En effet, celui-ci recouvrant, par l'extérieur, le volume à reconstruire, il suffit de l'enlever une fois la ou les vis retirées.

Avantageusement, le dispositif de l'invention peut comprendre en outre, de manière optionnelle, des perforations au niveau crestal, pour permettre l'insertion d'implants dentaires lors de l'étape chirurgicale de la régénération osseuse guidée.

Un autre objet de l'invention se rapporte à un procédé de fabrication d'un dispositif de l'invention tel que défini précédemment. Le procédé mis en œuvre peut comprendre notamment une étape de construction dudit dispositif en fonction d'une représentation 3D du défaut osseux obtenue par une technique d'imagerie dento-maxillaire, par exemple au moyen d'une technique de tomographie volumique à faisceaux coniques (Cône beam).

Avantageusement, et comme indiqué précédemment, la représentation 3D du défaut osseux peut être schématisée numériquement sur un logiciel adapté pour quantifier la substance osseuse à régénérer.

Eventuellement, la schématisation numérique permet d'obtenir un schéma, à partir duquel le dispositif de l'invention va être fabriqué de manière à recouvrir la représentation 3D du défaut osseux. Eventuellement, il est possible d'ajouter une couche de 1mm d'épaisseur supplémentaire, de manière à augmenter le volume à couvrir et obtenir un volume reconstruit supérieur, et à pallier à une formation d'une couche épithéliale superficielle.

Avantageusement, l'aspect lisse de la paroi du dispositif peut être obtenu par toute méthode connue de l'homme du métier. Il peut s'agir par exemple d'une étape d'impression 3D, ou d'usinage, ou encore de polissage, exemple au moyen de cupules adaptées.

Un autre objet de l'invention se rapporte à un procédé de régénération osseuse comprenant les étapes de :
- mise en place d'un dispositif selon l'invention au niveau d'un défaut osseux d'un patient,
- introduction de biomatériau dans le dispositif,
- pose du dispositif pendant un temps suffisant à la condensation du biomatériau, et
- retrait du dispositif de l'invention.

D'autres avantages pourront encore apparaître à l'homme du métier à la lecture des exemples ci-dessous, illustrés par les figures annexées, donnés à titre illustratif.

### Brève description des figures

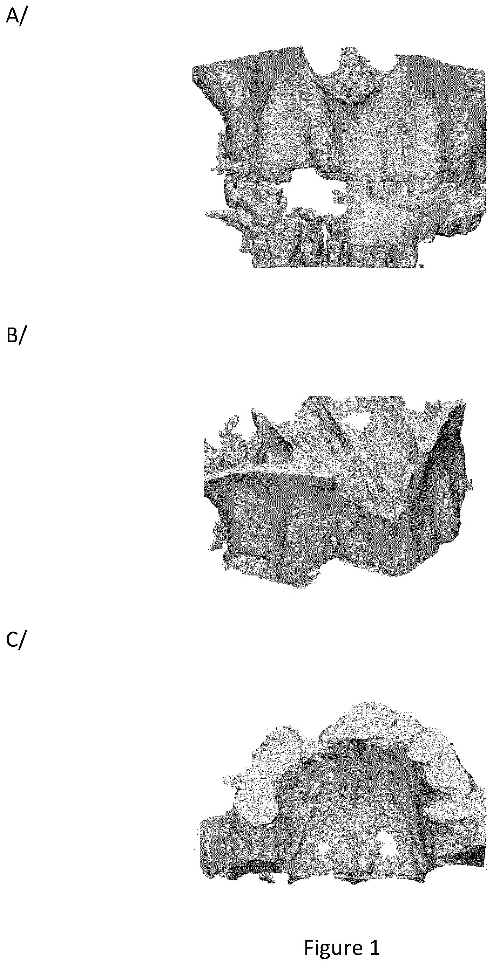

- La figure 1 représente une reconstruction volumique osseuse schématisée en 3 dimensions. A : vue de face du volume à reconstruire. B : vue du dessus du volume à reconstruire. C : vue du dessous du volume à reconstruire.

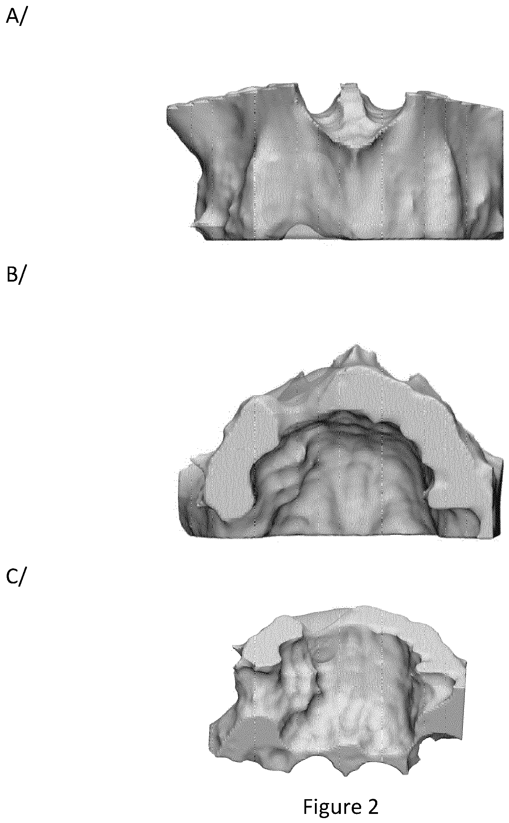

- La figure 2 représente une vue du volume osseux à reconstruire après nettoyage et définition du volume de comblement par effet miroir. A : vue de face du volume à reconstruire. B : vue du dessus du volume à reconstruire. C : vue du dessous du volume à reconstruire.

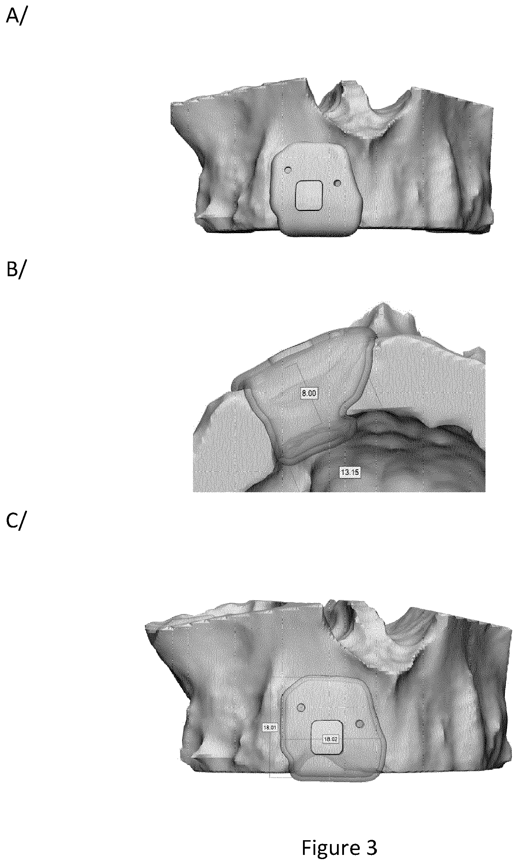

- La figure 3 représente une vue d'une coque d'épaisseur 0,8mm avec un décalage de 1mm au-dessus du volume à combler. A: vue de face du volume à reconstruire. B : vue du dessus du volume à reconstruire. C : vue de face du volume à reconstruire.

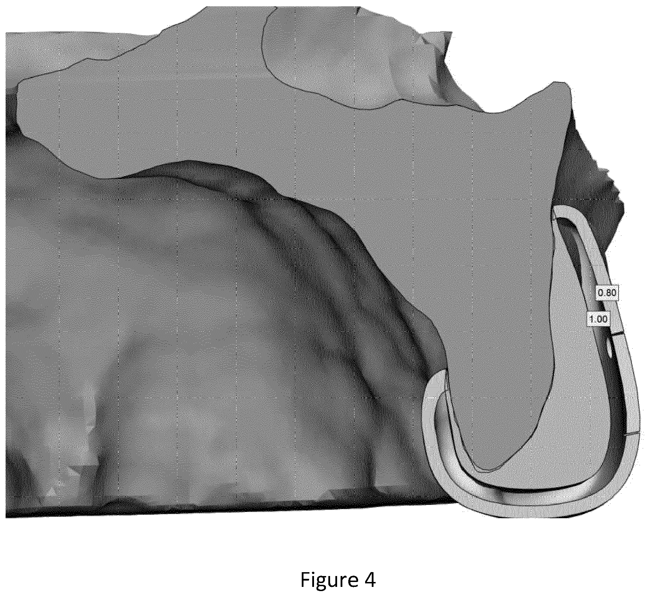

- La figure 4 représente une vue en coupe d'une coquille recouvrant le volume osseux à reconstruire.

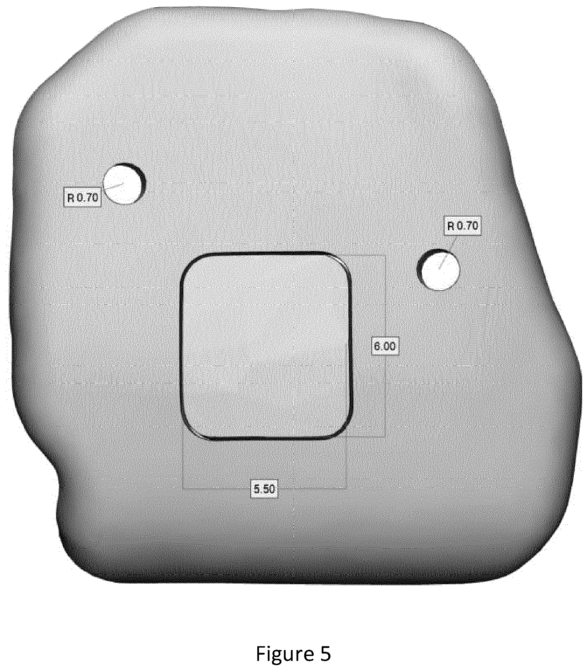

- La figure 5 représente une vue de face d'une coquille, comportant une fenêtre de 6 mm de haut et de 5,5 mm de large, et deux trous de fixation d'un diamètre de 1,4 mm chacun.

### EXEMPLES

### Exemple 1 : préparation d'un dispositif destiné à une régénération osseuse guidée en forme de coquille

On réalise une modélisation du volume de la quantité osseuse à reconstruire au moyen d'une tomographie volumique à faisceaux coniques (Cône beam).

Une représentation 3D de la reconstruction du défaut osseux est schématisée numériquement sur le logiciel MIMICS/3-matic, permettant de quantifier la substance osseuse à régénérer.

Grâce à ce schéma, une couche supplémentaire « optionnelle » de 1mm d'épaisseur est rajoutée à la représentation 3D, puis une coquille de 0,6 à 1,8 mm d'épaisseur est conçue de manière à couvrir la planification 3D évoquée.

Des perforations sont dessinées afin de stabiliser la future coquille de titane grâce à des vis d'ostéosynthèse.

Une fois le schéma de la coquille validé, la coquille est imprimée en titane au moyen d'une imprimante 3D. Le dispositif est ensuite poli afin que sa paroi soit totalement lisse.

### Exemple 2 : Exemple de mise en œuvre du dispositif de régénération osseuse guidée

Des médicaments préopératoires ont été prescrits au patient: acide amoxicilline clavulanique (2 g par jour), prednisolone (60 mg par jour).

Le paracétamol / codéine et le mouthwash (0,12 chlorexidine) ont été prescrits après l'opération.

La procédure a été réalisée sous anesthésie locale Ubistesine^{®} avec vasoconstricteur 1/200000.

Une décontamination à la Bétadine^{®} a été effectuée par voie intra et extra-buccale. Une incision est faite avec la lame du scalpel 15 C, supra-crestale puis intra-sulculaire pour se terminer avec deux incisions à libération buccale. Un lambeau de tissus complet a été levé, puis le lambeau est relâché après les incisions périostées. Le lambeau est disséqué à l'aide d'une paire de ciseaux Metzenbaum^{®}.

La maille / coque en titane personnalisée est positionnée. Le forage a été effectué à travers les trous de l'enveloppe existante. Les deux vis d'ostéosynthèse sont partiellement vissées, puis les biomatériaux allogéniques sont placés sous la coque. Les vis ont été serrées pour assurer la stabilité du biomatériau. Des sutures ont été réalisées, bord à bord, sans tension.

## Revendications

1. Dispositif de régénération osseuse guidée, destiné à la reconstruction d'un défaut osseux buccal, composé d'une paroi lisse en titane éventuellement microperforée, et ayant une forme recouvrant ledit défaut osseux buccal.

2. Dispositif selon la revendication 1, **caractérisé en ce que** le titane est pur, ou est un alliage composé de titane et d'au moins un autre élément chimique.

3. Dispositif selon la revendication 2, **caractérisé en ce que** ledit au moins un autre élément chimique est choisi parmi l'aluminium, le vanadium, le fer, l'afnium, le molybdène, l'oxygène, le palladium, l'étain, le niobium, le zirconium et le tantale.

4. Dispositif selon la revendication 2 ou 3, **caractérisé en ce que** ledit alliage est choisi parmi Ti-6Al-4V, Ti-6Al-7Nb, Ti-5Al-2,5Fe, Ti-13Ng-13Zr, Ti-12Mo-6Zr-2Fe, Ti-15Mo-5Zr-3Al, Ti-15Mo-3Nb-3O, Ti-15Zr-4Nb-2Ta-0.2Pd, Ti-15Sn-4Nb-2Ta-0.2Pd, Ti-35Nb-7Zr-5Ta, Ti-29Nb-13Ta-4,6Zr, Ti-35Nb-5Ta-7Zr-0,4O et Ti-Mo.

5. Dispositif selon l'une des revendications précédentes, **caractérisé en ce qu'**il possède une forme choisie parmi une coquille, une plaque et un filet.

6. Dispositif selon la revendication 5, **caractérisé en ce que** ledit dispositif est alvéolé ou non alvéolée.

7. Dispositif selon l'une quelconque des revendications précédentes, comprenant au moins une perforation de stabilisation dudit dispositif, ladite perforation étant destinée à recevoir une vis d'ostéosynthèse.

8. Dispositif selon l'une quelconque des revendications précédentes, dans lequel les microperforations sont disposées sur tout ou partie de la paroi.

9. Dispositif selon l'une quelconque des revendications précédentes, comprenant au moins une fenêtre.

10. Dispositif selon la revendication 9, dans lequel ladite au moins une fenêtre dont les dimensions sont inscrites dans un rectangle de 1 à 20 mm de haut et de 1 à 30 mm de large.

11. Dispositif selon l'une quelconque des revendications précédentes, ayant une épaisseur comprise entre 0,6 mm et 1,8 mm.

12. Procédé de fabrication d'un dispositif tel que défini dans l'une quelconque des revendications 1 à 11, comprenant une étape de construction dudit dispositif en fonction d'une représentation 3D obtenue par une technique d'imagerie dento-maxillaire du défaut osseux.

13. Procédé selon la revendication 12, dans lequel ladite représentation 3D dudit défaut osseux est schématisée numériquement sur un logiciel adapté pour quantifier la substance osseuse à régénérer.

14. Procédé selon l'une des revendications 12 ou 13, dans lequel ladite technique d'imagerie médicale est une technique de tomographie volumique à faisceaux coniques (Cône beam).

15. Procédé selon l'une quelconque des revendications 12 à 14, dans lequel ladite schématisation numérique permet d'obtenir un schéma, sur lequel une couche supplémentaire optionnelle de 1mm d'épaisseur est ajoutée.
